# EUROPEAN PATENT APPLICATION

(11) **EP 4 722 337 A1**
(43) Date of publication of application: **08.04.2026**
(21) Application number: 24815424.7
(22) Date of filing: 24.05.2024
(51) Int. Cl.: C12N 1/21, C12P 7/42, C12N 9/02, C12N 9/88, C12N 15/53, C12N 15/60

(54) **HYDROGENOPHILUS BACTERIUM TRANSFORMANT CAPABLE OF PRODUCING PROTOCATECHUIC ACID**

(30) Priority: 01.06.2023 JP 2023090701
(71) Applicant: Utilization of Carbon Dioxide Institute Co.,Ltd., Tokyo 135-0091 (JP)
(72) Inventor: YUKAWA, Hideaki, Tokyo 135-0091 (JP)
(74) Representative: Vossius & Partner Patentanwälte Rechtsanwälte mbB
(86) International application number: PCT/JP2024/019296
(87) International publication number: WO 2024/247941

(57) **Abstract**

A transformant obtained by introducing a chorismate-pyruvate lyase gene described in (a), (b), (c), (d), or (e) below and a 4-hydroxybenzoate hydroxylase gene described in (f), (g), (h), (i), or (j) below into a *Hydrogenophilus* bacterium is capable of efficiently producing protocatechuic acid utilizing substantially only carbon dioxide as a carbon source.
(a) a DNA comprising the nucleotide sequence of SEQ ID NO: 1
(b) a DNA comprising a nucleotide sequence having 90% or more identity with SEQ ID NO: 1 and encoding a polypeptide having chorismate-pyruvate lyase activity
(c) a DNA encoding a polypeptide comprising the amino acid sequence of SEQ ID NO: 2
(d) a DNA encoding a polypeptide comprising an amino acid sequence having 90% or more identity with SEQ ID NO: 2 and having chorismate-pyruvate lyase activity
(e) a DNA encoding a polypeptide comprising an amino acid sequence in which 1 to 10 amino acids are deleted, substituted, inserted, or added in the amino acid sequence of SEQ ID NO: 2, and having chorismate-pyruvate lyase activity
(f) a DNA comprising the nucleotide sequence of SEQ ID NO: 3
(g) a DNA comprising a nucleotide sequence having 90% or more identity with SEQ ID NO: 3 and encoding a polypeptide having 4-hydroxybenzoate hydroxylase activity
(h) a DNA encoding a polypeptide comprising the amino acid sequence of SEQ ID NO: 4
(i) a DNA encoding a polypeptide comprising an amino acid sequence having 90% or more identity with SEQ ID NO: 4 and having 4-hydroxybenzoate hydroxylase activity
(j) a DNA encoding a polypeptide comprising an amino acid sequence in which 1 to 30 amino acids are deleted, substituted, inserted, or added in the amino acid sequence of SEQ ID NO: 4, and having 4-hydroxybenzoate hydroxylase activity

## Description

### Technical Field

The present invention relates to a *Hydrogenophilus* bacterium transformant having ability to produce protocatechuic acid, and to a method for producing protocatechuic acid using the same.

### Background Art

The Paris Agreement, which was adopted in 2015, provides that global emissions of greenhouse gases should be promptly reduced. The Sixth Assessment Report published in 2023 by the Intergovernmental Panel on Climate Change (IPCC) states that if greenhouse gas emissions continue at high levels, there is a very high likelihood that the global average temperature will rise by more than 1.5°C compared to the pre-industrial era during the period from 2021 to 2040, and even if the emissions are kept low, the possibility of exceeding 1.5°C remains. The report also indicates that there would be a clear difference in climate change arisen across regions of the world between in the case where the temperature increase is limited to 1.5°C and in the case where the temperature reaches 2°C. Based on this, the report asserts that carbon neutrality must be achieved, that is, the global net anthropogenic CO₂ emissions must be reduced by approximately 45% by 2030 compared to 2010 levels and must reach net zero around 2050. In accordance with this report, the significant reduction of greenhouse gas emissions, including carbon dioxide and methane, has become a shared global challenge, thereby increasing the necessity for the development of relevant technologies.

The materials currently supplied by the chemical industry are indispensable for maintaining the present standards of science, culture, and daily life around the world, and a continuous and stable supply is required. However, the majority of chemical production globally still relies on petroleum-based raw materials, which contributes to the increase in greenhouse gas emissions. Accordingly, chemical manufacturing methods are expected to shift from conventional processes that heavily consume petroleum resources to carbon recycling-based methods that make effective use of carbon resources. To this end, research and development of biorefineries, which produce green chemicals from biomass using microbial fermentation, is being actively pursued in various countries. However, the saccharification of biomass for use as raw materials of microbial fermentation necessitates complex processes, and therefore, be costly. Using biomass that could serve as food or feed for chemical production disturbs the stable supply of food and feed. Furthermore, the large-scale consumption of biomass for chemical manufacturing raises concerns that it actually leads to environmental destruction.

In carbon recycling aimed at reducing fossil fuel consumption and greenhouse gas emissions, gases such as carbon dioxide, methane, and carbon monoxide are attracting attention as carbon sources having a higher level of sustainability. If these gases present in the atmosphere can be fixed by microorganisms and utilized for industrial purposes, it would significantly contribute to carbon recycling and support the achievement of the targeted carbon neutrality. Thus, techniques for producing valuable chemicals and biofuels using microorganisms that grow by utilizing these gases are the subject of interest. In particular, carbon fixation and efficient utilization of carbon dioxide, a significant contributor to global warming, is highly anticipated.

Protocatechuic acid is a valuable compound that serves as a component or raw material in the production of pharmaceuticals, cosmetics, agrochemicals, animal feed, and fragrances. Further, it is a useful compound that functions as an ingredient in functional foods due to its properties as a polyphenol with antioxidant activity. Furthermore, it is useful as a precursor for the biosynthetic or chemical production of valuable chemicals such as catechol and cis, cis-muconic acid, and as a raw material for high-performance polymers.

Conventionally, protocatechuic acid has been produced either by extraction from natural sources such as medicinal plants or by chemical synthesis from petroleum-derived materials. However, extraction from natural sources is costly due to low yields, and chemical synthesis from petroleum feedstocks imposes a high environmental burden.

In order to transition from a petroleum-dependent society, which faces concerns over future depletion of petroleum, toward a sustainable society, the production of protocatechuic acid by microbial fermentation using renewable, non-edible biomass resources has been attracting attention. However, as mentioned above, the use of biomass involves various challenges, including the need for complex processes, interference with the stable supply of food and feed, and significant environmental burden.

Accordingly, there is a demand for a practical method of producing protocatechuic acid using microorganisms that does not interfere with the stable supply of food and feed, not imposing environmental burden, and enables production through simpler processes. In particular, there is a need for a practical method that can produce protocatechuic acid by fixing carbon dioxide.

In many microorganisms, protocatechuic acid is produced from 4-hydroxybenzoic acid (4-HBA) via the catalytic activity of 4-hydroxybenzoate hydroxylase. Protocatechuic acid is also produced from 3-dehydroshikimic acid, which is part of the shikimate pathway involved in the synthesis of aromatic amino acids and other compounds, through the catalytic action of 3-dehydroshikimate dehydratase.

As a technique for producing protocatechuic acid by fermentation using genetically engineered microorganisms, Patent Literature 1, Non-Patent Literature 1, and Non-Patent Literature 2 disclose a method in which a chorismate-pyruvate lyase (*ubiC*) gene that produces 4-hydroxybenzoic acid from chorismic acid, a 4-hydroxybenzoate hydroxylase (*pobA*) gene that produces protocatechuic acid from 4-hydroxybenzoic acid, and a 3-dehydroshikimate dehydratase (*qsuB*) gene that produces protocatechuic acid from 3-dehydroshikimic acid are introduced into a *Corynebacterium* bacterium, and protocatechuic acid is produced using glucose as a carbon source. In the methods described in these Literatures, as the chorismate-pyruvate lyase gene, a gene derived from *Providencia rustigianii* is used; as the 4-hydroxybenzoate hydroxylase gene, a gene derived from *Corynebacterium glutamicum* is used; and as the 3-dehydroshikimate dehydratase gene, a plurality of genes derived from bacteria such as *Corynebacterium glutamicum* are used.

Furthermore, Non-Patent Literature 2 teaches a method in which *qsuB* gene derived from *Corynebacterium glutamicum* is introduced into *Corynebacterium glutamicum,* and protocatechuic acid is produced using glucose and D-xylose as carbon sources.

However, these methods are those producing protocatechuic acid by cultivating microorganisms using sugars, which are costly to produce, and none of the methods are those producing protocatechuic acid using carbon dioxide as a carbon source.

### Citation List

### Patent Literature

[Patent Literature 1] JP 2017/169399 A

### Non-Patent Literatures

[Non Patent Literature 1] Metab Eng., 65:232-242(2021)
[Non Patent Literature 2] Biotechnol Bioeng., 118(11):4414-4427 (2021)

### Summary of Invention

### Technical Problem

The objective of the present invention is to provide a transformant of a *Hydrogenophilus* bacterium (In other words, a transformed *Hydrogenophilus* bacterium) that is capable of efficiently producing protocatechuic acid utilizing carbon dioxide as a sole carbon source, and a method for efficiently producing protocatechuic acid using this transformant.

### Solution to Problem

The inventor of the present invention has focused on *Hydrogenophilus* bacteria as bacteria capable of fixing carbon dioxide on an industrial scale for the purpose of avoiding the use of high cost raw materials such as sugars and contributing to global warming countermeasures. *Hydrogenophilus* bacteria are bacteria which grow by producing organic substances from carbon dioxide by utilizing hydrogen energy. The growth rate of such bacteria is generally extremely slow, however, the growth rate of *Hydrogenophilus* bacteria is fast, and their ability of fixing carbon dioxide is remarkably higher than that of plants and photosynthetic bacteria.

*Hydrogenophilus* bacteria do not have enzymes for producing protocatechuic acid as a metabolic intermediate. Therefore, it is necessary to introduce the gene of an enzyme which catalyzes a reaction producing protocatechuic acid to *Hydrogenophilus* bacteria in order to provide capability of producing protocatechuic acid on an industrial scale to *Hydrogenophilus* bacteria.

The inventor of the present invention has found that when a heterologous gene which is expressed within bacteria other than *Hydrogenophilus* bacteria is introduced into *Hydrogenophilus* bacteria, a functioning protein often is not produced or is insufficiently produced. It is generally not practical to divert a gene which can produce a substance within bacteria other than *Hydrogenophilus* bacteria to *Hydrogenophilus* bacteria in order to produce the substance.

Under such circumstances, the present inventor has examined genes predicted to encode chorismate-pyruvate lyase in the genomes of various microorganisms in order to obtain a chorismate-pyruvate lyase gene that can be expressed in *Hydrogenophilus* bacteria. The present inventor has finally found that chorismate-pyruvate lyase gene of *Shewanella algae* allows the expression of functioning chorismate-pyruvate lyase in *Hydrogenophilus* bacteria.

The present inventor has also examined genes predicted to encode 4-hydroxybenzoate hydroxylase in the genomes of various microorganisms in order to obtain a 4-hydroxybenzoate hydroxylase gene that can be expressed in *Hydrogenophilus* bacteria. The present inventor has finally found that 4-hydroxybenzoate hydroxylase gene of *Corynebacterium glutamicum* allows the expression of functioning 4-hydroxybenzoate hydroxylase in *Hydrogenophilus* bacteria.

Further, the present inventor has found that a transformant obtained by introducing both of these genes into a *Hydrogenophilus* bacterium is capable of efficiently producing protocatechuic acid using carbon dioxide as a sole carbon source.

The present invention has been completed on the basis of the above findings and provides the following transformant and method for producing protocatechuic acid.
[1] A transformant obtained by introducing a chorismate-pyruvate lyase gene described in (a), (b), (c), (d), or (e) below and a 4-hydroxybenzoate hydroxylase gene described in (f), (g), (h), (i), or (j) below into a *Hydrogenophilus* bacterium (i.e., a transformed bacterium).
   (a) a DNA comprising the nucleotide sequence of SEQ ID NO: 1
   (b) a DNA comprising a nucleotide sequence having 90% or more identity with SEQ ID NO: 1 and encoding a polypeptide having chorismate-pyruvate lyase activity
   (c) a DNA encoding a polypeptide comprising the amino acid sequence of SEQ ID NO: 2
   (d) a DNA encoding a polypeptide comprising an amino acid sequence having 90% or more identity with SEQ ID NO: 2 and having chorismate-pyruvate lyase activity
   (e) a DNA encoding a polypeptide comprising an amino acid sequence in which 1 to 10 amino acids are deleted, substituted, inserted, or added in the amino acid sequence of SEQ ID NO: 2, and having chorismate-pyruvate lyase activity
   (f) a DNA comprising the nucleotide sequence of SEQ ID NO: 3
   (g) a DNA comprising a nucleotide sequence having 90% or more identity with SEQ ID NO: 3 and encoding a polypeptide having 4-hydroxybenzoate hydroxylase activity
   (h) a DNA encoding a polypeptide comprising the amino acid sequence of SEQ ID NO: 4
   (i) a DNA encoding a polypeptide comprising an amino acid sequence having 90% or more identity with SEQ ID NO: 4 and having 4-hydroxybenzoate hydroxylase activity
   (j) a DNA encoding a polypeptide comprising an amino acid sequence in which 1 to 30 amino acids are deleted, substituted, inserted, or added in the amino acid sequence of SEQ ID NO: 4, and having 4-hydroxybenzoate hydroxylase activity
[2] The transformant according to the above [1], wherein the *Hydrogenophilus* bacterium is *Hydrogenophilus thermoluteolus.*
[3] A method for producing protocatechuic acid, comprising a step of culturing the transformant according to the above [1] or [2] using substantially only carbon dioxide as a carbon source.

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical products can be produced while suppressing global warming attributed to increased carbon dioxide emissions.

Protocatechuic acid is an industrially important aromatic compound. However, protocatechuic acid extracted from natural products is costly because of low yield. Further, chemical synthesis from petroleum feedstock is undesirable from the viewpoint of environmental protection. In contrast, the production of protocatechuic acid using carbon dioxide-fixing microorganisms can provide a solution to global warming caused by increased carbon dioxide emissions as well as to securing the supply of industrially necessary protocatechuic acid.

Bacteria that can grow by utilizing the chemical energy generated by the reaction of hydrogen with oxygen and by using carbon dioxide as a sole carbon source, can produce chemical products from a mixture of oxygen, hydrogen, and carbon dioxide gases as raw material. Therefore, the bacteria can achieve the efficient conversion of carbon dioxide into organic compounds and can be cultured in a simple culture medium. Typically, such bacteria grow slowly, but the growth rate of hydrogen-oxidizing bacteria, *Hydrogenophilus* bacteria is exceptionally high. The Journal of Mitsubishi Research Institute No. 34 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

According to the present invention, introducing a specific chorismate-pyruvate lyase gene and a specific 4-hydroxybenzoate hydroxylase gene into *Hydrogenophilus* bacteria enables the expression of functional chorismate-pyruvate lyase and 4-hydroxybenzoate hydroxylase within these bacteria to produce protocatechuic acid on an industrial scale.

As described above, *Hydrogenophilus* bacteria have an atypically remarkable carbon dioxide fixation ability among organisms having carbon dioxide fixation ability. Therefore, the present invention has established a pathway for the industrial-scale production of protocatechuic acid.

### Mode for Carrying Out the Invention

Hereinafter, the present invention will be described in detail.

### (1) Transformant capable of producing protocatechuic acid

The transformant of the present invention is a transformant obtained by introducing a chorismate-pyruvate lyase gene (hereinafter sometimes abbreviated as *"ubiC* gene") of *Shewanella algae,* or a homologue thereof and a 4-hydroxybenzoate hydroxylase gene (hereinafter sometimes abbreviated as *"pobA* gene")of *Corynebacterium glutamicum,* or a homologue thereof, into a host *Hydrogenophilus* bacterium. That is, the transformant of the present invention is a *Hydrogenophilus* bacterium transformant having an exogenous *ubiC* gene, such as the *ubiC* gene of *Shewanella algae,* or a homologue thereof and an exogenous *pobA* gene, such as the *pobA* gene of *Corynebacterium glutamicum,* or a homologue thereof.

The *ubiC* gene and *pobA* gene used in the present invention do not have to be known genes identified as a *ubiC* gene and a *pobA* gene, respectively and may be any DNAs encoding polypeptides having chorismate-pyruvate lyase activity and 4-hydroxynenzoate hydroxylase, respectively. The chorismate-pyruvate lyase activity refers to an enzymatic activity to produce 4-hydroxynenzoic acid from chorismic acid. The 4-hydroxynenzoate hydroxylase activity refers to an enzymatic activity to produce protocatechuic acid from 4-hydroxynenzoic acid.

In the present invention, the *ubiC* gene and pobA gene may be DNAs corresponding to a *ubiC* gene and a pobA gene each isolated from naturally occurring bacteria, respectively, or may be DNAs artificially synthesized using methods known to those skilled in the art.

It is sufficient that the transformant of the present invention has *Shewanella algae ubiC* gene, or a homologue thereof and *Corynebacterium glutamicum pobA* gene, or a homologue thereof. The transformant of the present invention encompasses a transformant having two or more of *Shewanella algae ubiC* gene or a homologue thereof and two or more of *Corynebacterium glutamicum pobA* gene or a homologue thereof. The transformant of the present invention also encompasses a transformant having these genes and other genes.

### Chorismate-pyruvate lyase gene (ubiC gene)

In the present invention, a DNA comprising (particularly consisting of) the nucleotide sequence of SEQ ID NO: 1 can be used as the *ubiC* gene. SEQ ID NO: 1 is the nucleotide sequence of *Shewanella algae ubiC* gene.

In the present invention, a DNA comprising (particularly consisting of) a nucleotide sequence having 90% or more, especially 95% or more, especially 98% or more, especially 99% or more identity with SEQ ID NO: 1 and encoding a polypeptide having chorismate-pyruvate lyase activity can also be used.

In the present invention, a DNA encoding a polypeptide comprising (particularly consisting of) the amino acid sequence of SEQ ID NO: 2 as the *ubiC* gene. SEQ ID NO: 2 is the amino acid sequence of *Shewanella algae* chorismate-pyruvate lyase.

A DNA encoding a polypeptide comprising (particularly consisting of) an amino acid sequence having 90% or more, especially 95% or more, especially 98% or more, especially 99% or more identity with SEQ ID NO: 2 and having chorismate-pyruvate lyase activity can also be used.

Furthermore, a DNA encoding a polypeptide comprising (particularly consisting of) an amino acid sequence in which 1 to 10, 1 to 5, 1 to 4, 1 to 3, 1 to 2, or 1 amino acids are deleted, substituted, inserted, or added in the amino acid sequence of SEQ ID NO: 2, and having chorismate-pyruvate lyase activity can also be used.

In the present invention, the fact that a test polypeptide has chorismate-pyruvate lyase activity is determined by reacting the test polypeptide with barium chorismate, and detecting increase in absorbance at 246nm derived from produced 4-hydroxybenzoic acid.

### 4-hydroxybenzoate hydroxylase gene (pobA gene)

In the present invention, a DNA comprising (particularly consisting of) the nucleotide sequence of SEQ ID NO: 3 can be used as the *pobA* gene. SEQ ID NO: 3 is the nucleotide sequence of *Corynebacterium glutamicum pobA* gene.

In the present invention, a DNA comprising (particularly consisting of) a nucleotide sequence having 90% or more, especially 95% or more, especially 98% or more, especially 99% or more identity with SEQ ID NO: 3 and encoding a polypeptide having 4-hydroxybenzoate hydroxylase activity can also be used.

In the present invention, a DNA encoding a polypeptide comprising (particularly consisting of) the amino acid sequence of SEQ ID NO: 4 as the *pobA* gene. SEQ ID NO: 4 is the amino acid sequence of *Corynebacterium glutamicum* 4-hydroxybenzoate hydroxylase.

A DNA encoding a polypeptide comprising (particularly consisting of) an amino acid sequence having 90% or more, especially 95% or more, especially 98% or more, especially 99% or more identity with SEQ ID NO: 4 and having 4-hydroxybenzoate hydroxylase activity can also be used.

Furthermore, a DNA encoding a polypeptide comprising (particularly consisting of) an amino acid sequence in which 1 to 30, 1 to 10, 1 to 5, 1 to 4, 1 to 3, 1 to 2, or 1 amino acids are deleted, substituted, inserted, or added in the amino acid sequence of SEQ ID NO: 4, and having 4-hydroxybenzoate hydroxylase activity can also be used.

In the present invention, the fact that a test polypeptide has 4-hydroxybenzoate hydroxylase activity is determined by reacting the test polypeptide with 4-hydroxybenzoic acid in the presence of NADPH, and detecting decrease in absorbance at 340nm. Since 4-hydroxybenzoate hydroxylase consumes NADPH when producing protocatechuic acid from 4-hydroxybenzoic acid, decrease in NADPH is detected using decrease in absorbance at 340nm as an index.

DNA sequences encoding a protein that includes the amino acid sequence of SEQ ID NO: 2 or 4 may contain various nucleotide substitutions within the coding region due to codon degeneracy, or may contain various nucleotide substitutions considering codon preferences in *Hydrogenophilus* bacteria, as long as the substitutions do not alter the amino acid sequence of the protein expressed from the coding region.

In the present invention, the identities of nucleotide sequences and amino acid sequences were calculated using GENETYX ver.17 (made by GENETYX Corporation).

In the present invention, a DNA comprising a nucleotide sequence having an identity of 90% or more and less than 100% with the nucleotide sequence of a certain DNA and encoding a polypeptide having the same type of activity as the polypeptide encoded by the certain DNA is referred to as the "homolog" of the certain DNA. A DNA encoding a polypeptide comprising an amino acid sequence having an identity of 90% or more and less than 100% with the amino acid sequence of a certain polypeptide encoded by a certain DNA and having the same type of activity as the certain polypeptide is referred to as the "homolog" of the certain DNA. A DNA encoding a polypeptide comprising an amino acid sequence in which 1 to 30 amino acids are deleted, substituted, inserted, or added in the amino acid sequence of a certain polypeptide encoded by a certain DNA and having the same type of activity as the certain polypeptide is referred to as the "homolog" of the certain DNA.

### Hydrogenophilus bacteria

Examples of *Hydrogenophilus* bacteria include *Hydrogenophilus thermoluteolus, Hydrogenophilus halorhabdus, Hydrogenophilus denitrificans, Hydrogenophilus hirschii, Hydrogenophilus islandicus, Hydrogenophilus thiooxidans, Hydrogenophilus* bacterium strain Mar3 (*Hydrogenophilus* sp. Mar3), and *Hydrogenophilus* bacterium strain Z1038 (*Hydrogenophilus* sp. Z1038). In particular, *Hydrogenophilus thermoluteolus* is preferable because its superior growth rate enables top-level carbon dioxide fixation among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977)) (It proliferates double per hour.). *Hydrogenophilus thermoluteolus* strain NBRC 14978 is internationally deposited under the Budapest Treaty, and is thus publicly available.

The hosts *Hydrogenophilus* bacteria may be bacteria isolated from nature or genetically modified bacteria derived from bacteria isolated from nature. Genetic modification may be intended, for example, to allow high expression of an introduced chorismate-pyruvate lyase gene and 4-hydroxybenzoate hydroxylase gene.

Such a genetic modification can be made, for example, by removing (curing) endogenous plasmids in *Hydrogenophilus* bacteria. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### Method for producing transformant

Next, methods for obtaining transformants by introducing *ubiC* gene and *pobA* gene into *Hydrogenophilus* bacteria are described.

The introduction of *ubiC* gene and *pobA* into *Hydrogenophilus* bacteria can be carried out using common methods for introducing exogenous genes into bacteria. The *ubiC* gene and *pobA* gene may be directly introduced into *Hydrogenophilus* bacteria. Alternatively, a vector for transformation such as a plasmid vector, viral vector, cosmid, fosmid, BAC, or YAC may be used. In the case where a vector is used, a vector into which the *ubiC* gene and *pobA* gene have been incorporated may be introduced into a *Hydrogenophilus* bacterium, alternatively, a vector into which the *ubiC* gene has been incorporated and a vector into which the *pobA* gene has been incorporated may be introduced into a *Hydrogenophilus* bacterium.

Vectors for transformation should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), vectors obtained by genetically modifying these vectors (For example, pCAMO-6), and the like.

Among them, pCAMO-6 is preferable. Those skilled in the art can prepare pCAMO-6 according to "Examples" of this specification.

Examples of promoters in vectors include tac promoter, lac promoter, trc promoter, or each of promoters OXB1 and OXB11 to OXB20 from Oxford Genetics Ltd. Examples of terminators in vectors include the T1T2 terminator of *Escherichia coli* rRNA operon rrnB, the t0 transcription terminator of bacteriophage λ, T7 terminator, and the like.

Introduction of *ubiC* gene and *pobA* gene into a *Hydrogenophilus* bacterium (transformation) can be carried out by publicly known methods such as calcium chloride method, calcium phosphate method, rubidium chloride method, and electric pulse method (electroporation method).

### (2) Method for producing protocatechuic acid

The present invention provides a method for producing protocatechuic acid with use of the transformant of the present invention described above. The method includes a step of culturing the transformant of the present invention using an inorganic or organic culture medium while supplying a gas containing carbon dioxide, preferably a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that protocatechuic acid can be produced efficiently.

*Hydrogenophilus* bacteria can grow using hydrogen as a source of energy and using carbon dioxide as a sole carbon source, and thus, carbon dioxide can be fixed efficiently when protocatechuic acid is produced by cultivating the transformant of the present invention using substantially only carbon dioxide (in particular, by using only carbon dioxide) as a carbon source. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using only carbon dioxide as a carbon source" encompasses cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and protocatechuic acid can be produced efficiently.

When batch culture is utilized, mixed gases can be entrapped within an airtight culture container and static culture or shaking culture can be carried out. When continuous culture is utilized, mixed gases can be continuously supplied into an airtight culture container and shaking culture can be carried out, or the transformant can be cultured using an airtight culture container while introducing mixed gases into the culture medium by bubbling. Shaking culture is preferable in that better dissolution of mixed gases into the culture medium can be achieved.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and protocatechuic acid can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and protocatechuic acid can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and protocatechuic acid can be produced efficiently.

Protocatechuic acid or a salt thereof is produced in the medium solution by culturing the transformant in the above-described manner.

### Examples

The present invention will now be described with reference to examples. The technical scope of the present invention is not limited by the following examples.

### (1) Construction of plasmid vector (pCAMO-6)

The method for constructing a pCAMO-6 plasmid vector used for the introduction of *ubiC* gene and *pobA* gene is described below.

### (1-1) Preparation of tac promoter-containing DNA fragment

A pMAL-c5X plasmid manufactured by New England Biolabs was used as a template to amplify a DNA fragment containing a tac promoter by PCR using a pair of primers shown below. PCR was performed in the usual manner using 2720 Thermal Cycler manufactured by Thermo Fisher Scientific Inc. and using KOD FX Neo (manufactured by Toyobo Co., Ltd.) as a reaction reagent.
Primers for amplification of tac promoter
(a-1) 5'-TTTTATAACCCGGGCCATCGACTGCACGGTGCACC-3' (SEQ ID NO: 5)
(b-1) 5'-TGCTAGCACTGTTTCCTGTGTGAAATTGTTATCCG-3' (SEQ ID NO: 6)

The primer (a-1) contains a SmaI restriction enzyme site as indicated by underlining.

The resulting reaction mixture was subjected to electrophoresis on a 1% agarose gel. The results showed that an about 0.3-kbp DNA fragment corresponding to the tac promoter was detected.

### (1-2) Preparation of multicloning site-containing DNA fragment

For the preparation of a DNA fragment containing a multicloning site, the former and latter halves of the multicloning site were separately produced by PCR using pairs of primers shown below. PCR was performed in the usual manner using DNA Thermal Cycler manufactured by Life Technologies Inc. and using KOD FX Neo (manufactured by Toyobo Co., Ltd.) as a reaction reagent. This method does not use any template DNA and allows each pair of primers to anneal together in their 3' terminal regions and elongate to form a double-stranded DNA.
Primers for preparation of former half of multicloning site
(a-2) 5'-GGAAACAGTGCTAGCAGATCTGGAGGAGAAAGGCATAT-3' (SEQ ID NO: 7)

(b-2) 5′-
CAGTGCGGCCGCAAGCTTGTCGACGGAGCTCGAATTCGGATCCGATATCAGCATATGCGTTTC
TCCTCCAGA-3′ (SEQ ID NO: 8)

The 3' terminal nucleotide sequences of the primers (a-2) and (b-2) are complementary to each other.
Primers for preparation of latter half of multicloning site

(a-3) 5′-
ACAAGCTTGCGGCCGCACTGCAGCACCATCACCACCATCATTGATAAGATCCGGCTGCTAACA
AAGCCCGAAAGGAAGCT-3′ (SEQ ID NO: 9)

(b-3) 5′-
TATTTGAATCGAGTTATTGCTCAGCGGTGGCAGCAGCCAACTCAGCTTCCTTTCGGGCTTTGT
-3′ (SEQ ID NO: 10)

The 3' terminal nucleotide sequences of the primers (a-3) and (b-3) are complementary to each other.

The resulting reaction mixture was subjected to electrophoresis on a 1% agarose gel. The results showed that about 0.1-kbp DNA fragments corresponding to the former and latter halves of the multicloning site were detected. Each gel portion containing the DNA fragment was cut out of the agarose gel, and the gel portion was freeze-thawed to collect the DNA fragment.

Overlap extension PCR was performed through use of the collected DNA fragments corresponding to the former and latter halves of the multicloning site as a template. The 5' terminal nucleotide sequences of the primers (b-2) and (a-3) used to amplify these template DNA fragments are complementary to each other. For the overlap extension PCR, a combination of the primers (a-2) and (b-3) shown above was used to construct a DNA containing the multicloning site. PCR was performed in the usual manner using DNA Thermal Cycler manufactured by Life Technologies Inc. and using KOD FX Neo (manufactured by Toyobo Co., Ltd.) as a reaction reagent.

The resulting reaction mixture was subjected to electrophoresis on a 1% agarose gel. The results showed that an about 0.2-kbp DNA fragment corresponding to the multicloning site was detected.

### (1-3) Preparation of DNA fragment containing rrnB terminator joined to origin of replication of pUC19

Plasmid pMAL-c5X manufactured by New England Biolabs was used as a template to amplify a DNA fragment containing rrnB terminator by PCR using a pair of primers shown below.
Primers for amplification of rrnB terminator
(a-4) 5'-TAACTCGATTCAAATAAAACGAAAGGCTCAGTCGA-3' (SEQ ID NO: 11)
(b-4) 5'-CCTAGATCCGCGGAGTTTGTAGAAACGCAAAAAGG-3' (SEQ ID NO: 12)

In addition, pUC19 plasmid was used as a template to amplify a DNA fragment containing an origin of DNA replication by PCR using a pair of primers shown below.
Primers for amplification of origin of DNA replication of pUC19
(a-5) 5'-ACAAACTCCGCGGATCTAGGTGAAGATCCTTTTTG-3' (SEQ ID NO: 13)
(b-5) 5'-CGTCCGCGGCCAGCAAAAGGCCAGGAACCGTAAAA-3' (SEQ ID NO: 14)

Each PCR was performed in the usual manner using DNA Thermal Cycler manufactured by Life Technologies Inc. and using KOD FX Neo (manufactured by Toyobo Co., Ltd.) as a reaction reagent.

Each resulting reaction mixture was subjected to electrophoresis on a 1% agarose gel. The results showed that an about 0.3-kbp DNA fragment corresponding to the rrnB terminator and an about 0.8-kbp DNA fragment corresponding to the origin of DNA replication of pUC19 were detected. Each gel portion containing the DNA fragment was cut out of the agarose gel, and the gel portion was freeze-thawed to collect the DNA fragment.

Overlap extension PCR was performed through use of the collected DNA fragments corresponding to the rrnB terminator and the origin of DNA replication of pUC19 were used as templates. The 5' terminal nucleotide sequences of the primers (b-4) and (a-5) used to amplify these template DNA fragments are complementary to each other. For the overlap extension PCR, a combination of the primers (a-4) and (b-5) was used to construct a DNA fragment containing the rrnB terminator joined to the origin of replication of pUC19. PCR was performed in the usual manner using a DNA Thermal Cycler manufactured by Life Technologies Inc. and using a KOD FX Neo (manufactured by Toyobo Co., Ltd.) as a reaction reagent.

The resulting reaction mixture was subjected to electrophoresis on a 1% agarose gel. The results showed that an about 1.0-kbp DNA fragment corresponding to a DNA containing the rrnB terminator joined to the origin of replication of pUC19 was detected.

### (1-4) Preparation of DNA fragment containing neomycin/kanamycin resistance gene

Plasmid pK18mobsacB (GenBank: FJ437239.1) (Gene, 145, 69-73 (1994)), which contains a neomycin/kanamycin resistance gene (hereinafter sometimes referred to as "nptII") sequence, was used as a template for conventional PCR. For the amplification of a DNA fragment containing the nptII gene sequence, a pair of primers shown below was used for PCR. PCR was performed in the usual manner using DNA Thermal Cycler manufactured by Life Technologies Inc. and using KOD FX Neo (manufactured by Toyobo Co., Ltd.) as a reaction reagent.
Primers for amplification of nptII gene
(a-6) 5'-TTGCTGGCCGCGGACGTAGAAAGCCTGTCCGCAGA-3' (SEQ ID NO: 15)
(b-6) 5'-GGCCCGGGTTATAAAAGCCAGTCATTAGGCCTATC-3' (SEQ ID NO: 16)

The primer (b-6) contains a SmaI restriction enzyme site as indicated by underlining.

The resulting reaction mixture was subjected to electrophoresis on a 1% agarose gel. The results showed that an about 1.0-kbp DNA fragment corresponding to the nptII gene was detected.

### (1-5) Construction of circular plasmid

For the DNA fragments prepared in the above (1-1) to (1-4), the terminal region of the DNA fragment in the above (1-1) has a 16-bp sequence homologous to the terminal regions of the DNA fragments in the above (1-4) and (1-2); the terminal region of the DNA fragment in the above (1-2) has a 16-bp sequence homologous to the terminal regions of the DNA fragments in the above (1-1) and (1-3); the terminal region of the DNA fragment in the above (1-3) has a 16-bp sequence homologous to the terminal regions of the DNA fragments in the above (1-2) and (1-4); and the terminal region of the DNA fragment in the above (1-4) has a 16-bp sequence homologous to the terminal regions of the DNA fragments in the above (1-3) and (1-1).

Thus, the DNA fragments in the above (1-1), (1-2), (1-3), and (1-4) can be joined together into the form of a circular DNA by Gibson Assembly. Gibson Assembly was performed using Gibson Assembly Master Mix manufactured by New England Biolabs to join the DNA fragments prepared in the above (1-1) to (1-4) into a circular DNA. With the resulting reaction mixture, *Escherichia coli* JM109 was transformed according to calcium chloride method, and applied to a solid LB medium containing 50 µg/mL kanamycin. The grown strain on the medium was cultured in liquid medium in the usual manner. Plasmid DNA was extracted from the culture liquid and reacted with a restriction enzyme, SmaI. This reaction mixture was subjected to electrophoresis on a 1% agarose gel. The results showed that an about 2.3-kbp DNA fragment was observed, which corresponds in size to a single piece of DNA formed by joining the DNA fragments prepared in the above (1-1) to (1-4), demonstrating successful assembly of the DNA fragments.

This circular plasmid DNA, which is replicable in *Escherichia coli,* was named pCAMO-1.

### (1-6) Construction of plasmid vector (pCAMO-4)

*Hydrogenophilus thermoluteolus* TH-1 strain was inoculated with a platinum loop into 5 mL of liquid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, and 4.0 mg of CoCl₂·6H₂O were dissolved in 1 L of distilled water (pH 7.0)]in a test tube. The test tube was filled with a gas mixture of H₂:O₂:CO₂ = 7.5:1:1.5, and the strain was cultured at 50°C.

Endogenous plasmid, pTH1 (Microbiol Resour Announc. 2018 Aug 16; 7(6)), was prepared from the culture liquid according to the conventional alkaline-SDS method.

The prepared pTH-1 of about 66 kbp in size was cut with restriction enzymes ScaI and PvuII, and the pCAMO-1 plasmid was cut with restriction enzyme SmaI. Both of them were ligated together by T4 DNA (manufactured by Takara Bio Inc.).

With the resulting ligation mixture, *Hydrogenophilus thermoluteolus* TH-1 strain (NBRC 14978) was transformed according to electric pulse method (electroporation), applied to solid A medium [3.0 g of (NH₄)₂SO₄, 1.0 g of KH₂PO₄, 2.0 g of K₂HPO₄, 0.25 g of NaCl, 0.014 g of FeSO₄·7H₂O, 0.5 g of MgSO₄·7H₂O, 0.03 g of CaCl₂, 4.0 mg of MoO₃, 28 mg of ZnSO₄·7H₂O, 2.0 mg of CuSO₄·5H₂O, 4.0 mg of H₃BO₃, 4.0 mg of MnSO₄·5H₂O, 4.0 mg of CoCl₂·6H₂O, and 15 g of agar were dissolved in 1 L of distilled water (pH 7.0)] containing 50 µg/mL kanamycin, and cultured in a chamber filled with a gas mixture of H₂:O₂:CO₂ = 7.5:1:1.5 at 50°C for 60 hours.

Each of seven strains grown on the solid A medium was inoculated with a platinum loop into 5 mL of liquid A medium containing 50 µg/mL kanamycin in a test tube. The test tubes were filled with a gas mixture of H₂:O₂:CO₂ = 7.5:1:1.5, and the strains were cultured with shaking at 50°C. Plasmid DNA was extracted from the culture and subjected to electrophoresis on a 1% agarose gel. The results showed that all of the 7 strains had the same plasmid DNA of about 5.5 kbp in size.

The extracted plasmid was used as a template to amplify a DNA fragment containing the endogenous plasmid pTH1 inserted in the SmaI restriction enzyme site of pCAMO-1 by PCR using a pair of primers shown below. The pair of primers correspond to the regions at both sides of the SmaI restriction enzyme site of pCAMO-1. PCR was performed in the usual manner using DNA Thermal Cycler manufactured by Life Technologies Inc. and using KOD FX Neo (manufactured by Toyobo Co., Ltd.) as a reaction reagent.
Primers for amplification of inserted pTH1 DNA fragment
(a-7) 5'-AATTGTCAGATAGGCCTAATGACTGGCTTTTATAA-3' (SEQ ID NO: 17)
(b-7) 5'-TGACGCCAGAAGCATTGGTGCACCGTGCAGTCGAT-3' (SEQ ID NO: 18)

The resulting reaction mixture was subjected to electrophoresis on a 1% agarose gel. The results showed that an about 3.2-kbp DNA fragment was detected. That is, the obtained plasmid contains an about 3.2-kbp DNA fragment corresponding to a part of pTH1, which has been inserted into the SmaI restriction enzyme site of the pCAMO-1 plasmid. This about 3.2-kbp DNA fragment contains the origin of replication that functions in *Hydrogenophilus thermoluteolus* cells, so that the obtained plasmid replicates in *Hydrogenophilus thermoluteolus* cells.

The constructed plasmid was named pCAMO-4.

### (1-7) Construction of plasmid vector (pCAMO-5)

Equal moles of a pair of oligonucleotides shown below were mixed, and the mixture was gently cooled from 98°C to 20°C, thus yielding a double-stranded DNA in which the oligonucleotides were annealed. Both ends of this DNA fragment are equivalent to the protruding ends generated by cleavage with restriction enzymes BglII and NdeI. This DNA fragment and a digested DNA fragment prepared by cutting pCAMO-4 with restriction enzymes BglII and NdeI were ligated together by T4 DNA ligase (manufactured by Takara Bio Inc.).
(a-8) 5'-GATCTGGAGGAGAAACGCA-3' (SEQ ID NO: 19)
(b-8) 5'-TATGCGTTTCTCCTCCA-3' (SEQ ID NO: 20)

The constructed plasmid was named pCAMO-5.

### (1-8) Construction of plasmid vector (pCAMO-6)

The endogenous plasmid pTH-1 was used as a template to amplify a DNA fragment containing a mazF gene encoding a plasmid stabilization factor by PCR using a pair of primers shown below. PCR was performed in the usual manner using DNA Thermal Cycler manufactured by Life Technologies Inc. and using KOD FX Neo (manufactured by Toyobo Co., Ltd.) as a reaction reagent.
Primers for amplification of mazF gene
(a-9) 5'-GAGGCCATCTAGGCCATGAGTAAGTCTGACGGAAC-3' (SEQ ID NO: 21)
(b-9) 5'-ATCCGGCACCCATATCTGAACCGGACGCAAACCCG-3' (SEQ ID NO: 22)

The endogenous plasmid pTH-1 was used as a template to amplify a DNA fragment containing a mazE gene encoding a plasmid stabilization factor by PCR using a pair of primers shown below. PCR was performed in the usual manner using DNA Thermal Cycler manufactured by Life Technologies Inc. and using KOD FX Neo (manufactured by Toyobo Co., Ltd.) as a reaction reagent.
Primers for amplification of mazE gene
(a-10) 5'-TTCAGATATGGGTGCCGGATACCCGCCGCCCGGGC-3' (SEQ ID NO: 23)
(b-10) 5'-AAGGCCTTCATGGCCTTATTTCGCGATTCCCAAGA-3' (SEQ ID NO: 24)

The 3' terminal region of the mazF-containing DNA fragment has a 20-bp sequence homologous to the 5' terminal region of the mazE-containing DNA fragment. Thus, the mazF-containing DNA fragment and the mazE-containing DNA fragment can be joined by PCR. The DNA fragments prepared above were mixed, and for the amplification of the joined DNA fragments, the primers (a-9) and (b-10) were used. PCR was performed in the usual manner using DNA Thermal Cycler manufactured by Life Technologies Inc. and using KOD FX Neo (manufactured by Toyobo Co., Ltd.) as a reaction reagent.

The resulting reaction mixture was subjected to electrophoresis on a 1% agarose gel. The results showed that an about 0.7-kbp DNA fragment corresponding to a DNA formed by joining the mazF-containing DNA fragment and the mazE-containing DNA fragment was detected.

The prepared DNA fragment of about 0.7 kbp in size was cut with restriction enzyme SfiI, and the pCAMO-5 plasmid was cut with restriction enzyme SfiI. Both fragments were ligated together by T4 DNA ligase (manufactured by Takara Bio Inc.).

The constructed plasmid was named pCAMO-6.

### (2) Construction of expression plasmid for ubiC gene

An expression plasmid for *ubiC* gene was constructed by, what is called, seamless cloning, a method for cloning a target DNA into a vector through recombination between homologous sequences. A DNA fragment of *ubiC* gene derived from *Shewanella algae* (SEQ ID NO: 1) was amplified by PCR

The primers shown below were used for PCR. PCR was performed in the usual manner using DNA Thermal Cycler manufactured by Life Technologies Inc. and using KOD One PCR Master Mix (manufactured by Toyobo Co., Ltd.) as a reaction reagent.
Primers for amplification of *Shewanella algae ubiC* gene
(a-11) 5'-GCAGATCTGGAGGAGAAACGCATATGAGTGTGACCAGTTTAAGC-3' (SEQ ID NO: 25)
(b-11) 5'-CTTGTCGACGGAGCTCGAATTCTCAAGGCGTAAGTTGGCCTTG-3' (SEQ ID NO: 26)

The primers (a-11) and (b-11) contain their respective sequences homologous to sequences in the pCAMO-6 vector.

The resulting reaction mixture was subjected to electrophoresis on a 1% agarose gel. The results showed that an about 0.7-kbp fragment corresponding to the chorismite-pyruvate lyase gene of the bacterial strain was detected.

A gel portion containing the DNA fragment corresponding to the *ubiC* gene was cut out of the agarose gel, and the DNA fragment containing the *ubiC* gene was collected from the gel portion with GEL/PCR Purification Mini Kit (FAVORGEN).

The pCAMO-6 plasmid vector was amplified by PCR for subsequent seamless cloning. The primers shown below were used for PCR. PCR was performed in the usual manner using DNA Thermal Cycler manufactured by Life Technologies Inc. and using KOD One PCR Master Mix (manufactured by Toyobo Co., Ltd.) as a reaction reagent.
Primers for amplification of pCAMO-6 plasmid vector
(a-12) 5'-GAATTCGAGCTCCGTCGACA-3' (SEQ ID NO: 27)
(b-12) 5'-ATGCGTTTCTCCTCCAGATC-3' (SEQ ID NO: 28)

The gel portion containing a DNA fragment corresponding to the vector gene was cut out of the agarose gel, and the DNA fragment containing the vector gene was collected from the gel portion with GEL/PCR Purification Mini Kit (FAVORGEN).

The DNA fragment containing the pCAMO-6 vector as synthesized above and the DNA fragment containing the *ubiC* gene were joined together by a recombinase extracted from *Escherichia coli* JM109 strain.

With the resulting reaction mixture, *Escherichia coli* JM109 strain was transformed by heat shock method, applied to LB media with 50 µg/mL kanamycin, and cultured at 37°C for 24 hours.

The strain grown on the LB media was inoculated with a platinum loop into 5 mL of liquid LB medium containing 50 µg/mL kanamycin in a test tube, and cultured with shaking at 37°C. Plasmid DNA was extracted from the culture liquid. The sequence of the *ubiC* gene inserted in the plasmid was analyzed according to Sanger method by Eurofins Genomics K.K. under contract, and confirmed to be identical to the corresponding sequence in the database.

### (3) Construction of expression plasmids for pobA genes

Expression plasmids for *pobA* genes were constructed by, what is called, seamless cloning, a method for cloning a target DNA into a vector through recombination between homologous sequences.

DNA fragments containing *pobA* genes from different bacteria shown below were amplified by PCR.
*Corynebacterium glutamicum* (SEQ ID NO: 3)
*Meiothermus timidus* (SEQ ID NO: 29)
*Amycolatopsis thermoflava* (SEQ ID NO: 30)

The primers shown below were used for PCR. PCR was performed in the usual manner using DNA Thermal Cycler manufactured by Life Technologies Inc. and using KOD One PCR Master Mix (manufactured by Toyobo Co., Ltd.) as a reaction reagent.
Primers for amplification of *Corynebacterium glutamicum pobA* gene
(a-13) 5'-CAGATCTGGAGGAGAAACGCATATGAACCACGTACCAGTGGC-3' (SEQ ID NO:31)
(b-13) 5'-CTTGTCGACGGAGCTCGAATTCTTATACCTCGAAGCGTGGTAG-3' (SEQ ID NO:32)

The primers (a-13) and (b-13) contain their respective sequences homologous to sequences in the pCAMO-6 vector.
Primers for amplification of *Meiothermus timidus pobA* gene
(a-14) 5'-CAGATCTGGAGGAGAAACGCATATGGCTAGGGTGGGCGTGGTG-3' (SEQ ID NO:33)
(b-14) 5'-GTCGACGGAGCTCGAATTCCTAGGCCGAGACCCCCTCCCCCA-3' (SEQ ID NO:34)

The primers (a-14) and (b-14) contain their respective sequences homologous to sequences in the pCAMO-6 vector.
Primers for amplification of *Amycolatopsis thermoflava pobA* gene
(a-15) 5'-GATCTGGAGGAGAAACGCATATGACGCGCACACAGGTCGGGATC-3' (SEQ ID NO:35)
(b-15) 5'-CTTGTCGACGGAGCTCGAATTCTTACGCATTACTCGTCCAGGCG-3' (SEQ ID NO:36)

The primers (a-15) and (b-15) contain their respective sequences homologous to sequences in the pCAMO-6 vector.

The resulting reaction mixtures were subjected to electrophoresis on a 1% agarose gel. The results showed that about 1.2-kbp DNA fragments for *pobA* genes of the respective bacterial strains were detected.

Each of gel portions containing the DNA fragment corresponding to the *pobA* gene was cut out of the agarose gel, and the DNA fragments containing the *pobA* genes were collected from the gel portions with GEL/PCR Purification Mini Kit (FAVORGEN).

The pCAMO-6 plasmid vector was amplified by PCR for subsequent seamless cloning. The primers shown below were used for PCR. PCR was performed in the usual manner using DNA Thermal Cycler manufactured by Life Technologies Inc. and using KOD One PCR Master Mix (manufactured by Toyobo Co., Ltd.) as a reaction reagent.
Primers for amplification of pCAMO-6 plasmid vector
(a-12) 5'-GAATTCGAGCTCCGTCGACA-3' (SEQ ID NO: 27)
(b-12) 5'-ATGCGTTTCTCCTCCAGATC-3' (SEQ ID NO: 28)

The gel portion containing a DNA fragment corresponding to the vector gene was cut out of the agarose gel, and the DNA fragment was collected from the gel portion with GEL/PCR Purification Mini Kit (FAVORGEN).

The DNA fragment containing the pCAMO-6 vector as synthesized above and the DNA fragment containing each *pobA* gene were joined together by a recombinase extracted from *Escherichia coli* JM109 strain.

With the resulting reaction mixtures, *Escherichia coli* JM109 strain was transformed by heat shock method, applied to LB media with 50 µg/mL kanamycin, and cultured at 37°C for 24 hours.

Each of the strains grown on the LB media was inoculated with a platinum loop into 5 mL of liquid LB medium containing 50 µg/mL kanamycin in a test tube, and cultured with shaking at 37°C. Plasmid DNA was extracted from the culture liquid. The sequence of the *pobA* gene inserted in each plasmid was analyzed according to Sanger method by Eurofins Genomics K.K. under contract, and confirmed to be identical to the corresponding sequence in the database.

### (4) Construction of plasmid for production of protocatechuic acid

A plasmid for production of protocatechuic acid was constructed by, what is called, seamless cloning, a method for cloning a target DNA into a vector through recombination between homologous sequences.

The above-mentioned expression plasmid including *ubiC* gene derived from *Shewanella algae* was amplified by PCR. Primers shown below were used for the PCR. PCR was performed in the usual manner using DNA Thermal Cycler manufactured by Life Technologies Inc. and using KOD One PCR Master Mix (manufactured by Toyobo Co., Ltd.) as a reaction reagent.
Primers for amplification of expression plasmid including *Shewanella algae ubiC* gene
(a-16) 5'-CCGCGGATCTAGGTGAAGATC-3' (SEQ ID NO: 37)
(b-16) 5'-AGTTTGTAGAAACGCAAAAAGGCCATC-3' (SEQ ID NO: 38)

The primers (a-16) and (b-16) contain their respective sequences homologous to sequences in the pCAMO-6 vector.

A gel portion containing corresponding to the vector gene including *Shewanella algae ubiC* gene was cut out of an agarose gel, and the DNA fragment was collected from the gel portion with GEL/PCR Purification Mini Kit (FAVORGEN).

The *pobA* gene including trc promotor and rrnB terminator was amplified based on the expression plasmid including the above-mentioned *pobA* gene derived from *Corynebacterium glutamicum.* Primers shown below were used for the PCR. PCR was performed in the usual manner using DNA Thermal Cycler manufactured by Life Technologies Inc. and using KOD One PCR Master Mix (manufactured by Toyobo Co., Ltd.) as a reaction reagent.
Primers for amplification of *pobA* gene including trc promotor and rrnB terminator
(a-17) 5'-GCCTTTTTGCGTTTCTACAAACTCCCATCGACTGCACGGTGCAC-3' (SEQ ID NO: 39)
(b-17) 5'-GTCATGAGATTATCAAAAAGGATCTTCACCTAGATCCGCG-3' (SEQ ID NO: 40)

The gel portion corresponding to the *pobA* gene containing trc promotor and rrnB terminator was cut out of the agarose gel, and the DNA fragment was collected from the gel portion with GEL/PCR Purification Mini Kit (FAVORGEN).

The DNA fragment containing the vector including *ubiC* gene derived from *Shewanella algae* and the DNA fragment containing the vector including *pobA* gene including trc promotor and rrnB terminator, as synthesized above, were joined together by a recombinase extracted from *Escherichia coli* JM109 strain.

With the resulting reaction mixture, *Escherichia coli* JM109 strain was transformed by heat shock method, applied to LB media with 50 µg/mL kanamycin, and cultured at 37°C for 24 hours.

The strain grown on the LB media was inoculated with a platinum loop into 5 mL of liquid LB medium containing 50 µg/mL kanamycin in a test tube, and cultured with shaking at 37°C. Plasmid DNA was extracted from the culture liquid. The sequences of the *ubiC* gene and *pobA* gene inserted in the plasmid was analyzed according to Sanger method by Eurofins Genomics K.K. under contract, and confirmed to be identical to the corresponding sequence in the database.

### (5) Transformants of Hydrogenophilus thermoluteolus

### (5-1) Introduction of expression vector for pobA

*Hydrogenophilus thermoluteolus* TH-1 strain was transformed with the obtained plasmids in the item "(3) Construction of expression plasmids for *pobA* genes" according to electric pulse method (electroporation), applied to solid LB media containing 50 µg/mL kanamycin, and cultured at 52°C for 24 hours. The strains grown on the solid LB media were inoculated with a platinum loop on separate solid LB media containing 50 µg/mL kanamycin, and cultured at 52°C for 24 hours. The grown strains on the solid LB media were examined to confirm whether the inserted fragments in the respective plasmids would be amplified by PCR. Each PCR was performed in the usual manner using DNA Thermal Cycler manufactured by Life Technologies Inc. and using KOD One PCR Master Mix (manufactured by Toyobo Co., Ltd.) as a reaction reagent.

The results showed that DNA fragments of about 1.2-kbp in size corresponding to the respective *pobA* genes were amplified. The plasmids containing the *pobA* genes of the respective bacteria and the *Hydrogenophilus thermoluteolus* TH-1 transformants with the respective plasmids were named as shown in Table 1.

### (5-2) Measurement of 4-hydroxybenzoate hydroxylase activity

The transformed strains of *Hydrogenophilus thermoluteolus* with the respective *pobA* genes as prepared above were inoculated with a platinum loop into separate liquid A media containing 50 µg/mL kanamycin, and cultured with shaking at 52°C for 24 hours while a gas mixture of H₂:O₂:CO₂ = 7.5:1:1.5 was supplied. The cultured bacterial cells were collected by centrifugation (4°C, 5,000 g, 10 minutes). The bacterial cells were disrupted by sonication and then centrifuged (4°C, 20,000 g, 3 minutes) to obtain the supernatants of the disrupted cells.

4-hydroxybenzoate hydroxylase activity was measured using the disrupted cell supernatants as crude enzyme solutions. That is, these crude enzyme solutions were separately mixed with 50mM tris-HCl (pH 7.5), 175µM NADPH, 65 µM FAD, and 0.5 mM 4-hydroxybenzoic acid for a reaction to proceed at 52°C. The decrease in absorbance at 340 nm derived from NADPH was monitored, and the initial rate of the reaction was analyzed. The specific activity of 4-hydroxybenzoate hydroxylase was calculated from the initial rate of the reaction (The specific activity of an enzyme producing 1µM protocatechuic acid per minute per milligram of total protein was defined as 1 unit). As shown in Table 1, 4-hydroxybenzoate hydroxylase activity was detected only in strain PobA01, in which the *pobA* gene from *Corynebacterium glutamicum* had been introduced.

**[Table 1]**

| Strain | Origin of 4-hydroxybenzoate hydroxylase gene | 4-hydroxybenzoate hydroxylase activity (U/mg protein) |
|---|---|---|
| PobA01 | *Corynebacterium glutamicum* | 0.8 |
| PobA02 | Meiothermus timidus | ND |
| PobA03 | *Amycolatopsis thermoflava* | ND |
| p-CAMO-6/TH-1 | None | ND |

| | | |
|---|---|---|
| 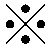 ND : Not Detected (below the detection limit) | | |

### (5-3) Introduction of plasmid for producing protocatechuic acid

*Hydrogenophilus thermoluteolus* TH-1 strain was transformed with the plasmid obtained in the item "(4) Construction of plasmid for production of protocatechuic acid" according to electric pulse method (electroporation), applied to solid LB media containing 50 µg/mL kanamycin, and cultured at 52°C for 24 hours. The strains grown on the solid LB media were inoculated with a platinum loop on separate solid LB media containing 50 µg/mL kanamycin, and cultured at 52°C for 24 hours. The grown strains on the solid LB media were examined to confirm whether the inserted fragment in the plasmid would be amplified by PCR. PCR was performed in the usual manner using DNA Thermal Cycler manufactured by Life Technologies Inc. and using KOD One PCR Master Mix (manufactured by Toyobo Co., Ltd.) as a reaction reagent.

The results showed that DNA fragment of about 3.2-kbp in size corresponding to *ubiC* and *pobA* genes joined with tac promoter and rrnB terminator were amplified.

### (5-4) Production of protocatechuic acid

The *Hydrogenophilus thermoluteolus* strain as prepared above to which the expression plasmid for producing protocatechuic acid was introduced was inoculated with a platinum loop into liquid A medium containing 50 µg/mL kanamycin, and cultured with shaking at 52°C for 72 hours while a gas mixture of H₂:O₂:CO₂ = 7.5:1:1.5 was supplied. The strain TH-1 having empty vector pCAMO-6 was similarly cultured. After that, culture supernatants were collected by centrifugation (4°C, 5,000 g, 10 minutes). The concentrations of protocatechuic acid in the culture supernatants were determined using a high-performance liquid chromatography (Shimadzu Corporation) under the following conditions.
Mobile phase A: 0.1% phosphoric acid aqueous solution
Mobile phase B: 50% acetonitrile
Flow rate: 1 mL/min
Column: CAPCELLPAK MGII, 5.0µm, 4.6mm I.D.×150mm
Column temperature: 40°C
PDA temperature: 40°C
PDA: 200-300nm
Reference wavelength: 210nm

As a result, 0.41 mM of protocatechuic acid was detected in the culture supernatant. On the other hand, in a strain into which neither the *ubiC* gene nor the *pobA* gene had been introduced, protocatechuic acid was not detected. By introducing the *ubiC* gene of *Shewanella algae* and the *pobA* gene of *Corynebacterium glutamicum* to the *Hydrogenophilus* bacterium, protocatechuic acid-producing capability was provided to the *Hydrogenophilus* bacterium.

The *Hydrogenophilus* bacterium transformant of the present invention can be prepared by referring to the description of "Examples" of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

### Industrial Applicability

The transformant of the present invention can highly efficiently produce protocatechuic acid using carbon dioxide as a sole carbon source, thus providing a solution to global warming caused by increased carbon dioxide emissions and contributing to high-efficient industrial production of protocatechuic acid and chemical products produced using protocatechuic acid as a raw material.

## Claims

1. A transformant obtained by introducing a chorismate-pyruvate lyase gene described in (a), (b), (c), (d), or (e) below and a 4-hydroxybenzoate hydroxylase gene described in (f), (g), (h), (i), or (j) below into a *Hydrogenophilus* bacterium.
(a) a DNA comprising the nucleotide sequence of SEQ ID NO: 1
(b) a DNA comprising a nucleotide sequence having 90% or more identity with SEQ ID NO: 1 and encoding a polypeptide having chorismate-pyruvate lyase activity
(c) a DNA encoding a polypeptide comprising the amino acid sequence of SEQ ID NO: 2
(d) a DNA encoding a polypeptide comprising an amino acid sequence having 90% or more identity with SEQ ID NO: 2 and having chorismate-pyruvate lyase activity
(e) a DNA encoding a polypeptide comprising an amino acid sequence in which 1 to 10 amino acids are deleted, substituted, inserted, or added in the amino acid sequence of SEQ ID NO: 2, and having chorismate-pyruvate lyase activity
(f) a DNA comprising the nucleotide sequence of SEQ ID NO: 3
(g) a DNA comprising a nucleotide sequence having 90% or more identity with SEQ ID NO: 3 and encoding a polypeptide having 4-hydroxybenzoate hydroxylase activity
(h) a DNA encoding a polypeptide comprising the amino acid sequence of SEQ ID NO: 4
(i) a DNA encoding a polypeptide comprising an amino acid sequence having 90% or more identity with SEQ ID NO: 4 and having 4-hydroxybenzoate hydroxylase activity
(j) a DNA encoding a polypeptide comprising an amino acid sequence in which 1 to 30 amino acids are deleted, substituted, inserted, or added in the amino acid sequence of SEQ ID NO: 4, and having 4-hydroxybenzoate hydroxylase activity

2. The transformant according to claim 1, wherein the *Hydrogenophilus* bacterium is *Hydrogenophilus thermoluteolus.*

3. A method for producing protocatechuic acid, comprising a step of culturing the transformant according to claim 1 or 2 using substantially only carbon dioxide as a carbon source.
